(19) Europäisches Patentamt
European Patent Office
Office européen des brevets

(11) Numéro de publication : **0 206 953**
**B1**

(12) **FASCICULE DE BREVET EUROPÉEN**

(45) Date de publication du fascicule du brevet : **07.09.88**

(51) Int. Cl.[4] : **C 07 C 67/343**, C 07 C 69/716

(21) Numéro de dépôt : **86420153.8**

(22) Date de dépôt : **13.06.86**

(54) **Procédé de préparation de trifluoroacétoacétate d'éthyle.**

(30) Priorité : **14.06.85 FR 8509023**

(43) Date de publication de la demande : **30.12.86 Bulletin 86/52**

(45) Mention de la délivrance du brevet : **07.09.88 Bulletin 88/36**

(84) Etats contractants désignés : **AT BE CH DE FR GB IT LI LU NL SE**

(56) Documents cités : **Eléments de la technique relevés: néant**

(73) Titulaire : **RHONE-POULENC CHIMIE**
**25, quai Paul Doumer**
**F-92408 Courbevoie Cédex (FR)**

(72) Inventeur : **Amiet, Louis**
**10, Quai Saint-Vincent**
**F-69001 Lyon (FR)**
Inventeur : **Langlois, Bernard**
**91, rue Duguesclin**
**F-69006 Lyon (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

**EP 0 206 953 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation de trifluoroacétoacétate d'éthyle. Elle concerne plus particulièrement un procédé de préparation par condensation de trifluoroacétate d'éthyle et d'acétate d'éthyle en présence d'une base.

Il est connu d'après SWARTS dans le « bulletin des Sciences académiques du Royaume de Belgique 5, 12, 679-725, (1926) » de condenser le trifluoracétate d'éthyle et l'acétate d'éthyle, en présence d'éthanolate de sodium. La condensation est réalisée en milieu éther anhydre à reflux et conduit à l'énolate de sodium. On libère par l'acide sulfurique l'énol, qui s'équilibre avec sa forme cétonique. Cette réaction en milieu aqueux, en présence d'un acide fort, provoque une hydrolyse partielle du b cétoester obtenu ce qui nuit fortement à la rentabilité du procédé.

Il est aussi connu d'après le Journal of Fluorine Chemistry [BAYER, PASTOR, CAMBON 20, 187-202 (1982)] de réaliser le même procédé que précédemment mais en remplaçant l'éthanolate de sodium par l'hydrure de sodium. Les rendements obtenus sont améliorés. Mais l'utilisation d'éther en grandes quantités et la neutralisation par un acide minéral aqueux rendent ce procédé comme le précédent, difficilement utilisable d'un point de vue industriel, à cause à la fois des mesures de sécurité indispensables et de la mauvaise rentabilité économique de ces procédés.

Certains auteurs ont essayé d'éviter l'emploi de l'éther lors de la condensation des esters éthyliques de l'acide trifluoracétique et acétique [BURDON, MAC LOUGHLIN, tétrahedron, 20, 2163-6 (1964)] mais la phase de libération de l'énol se fait toujours en milieu aqueux avec utilisation d'éther. Ces auteurs affirment eux-mêmes que le procédé est inapplicable à grande échelle.

Une technique réactionnelle tout à fait différente est décrite dans le brevet français N° 1310174. En effet, dans ce brevet, on décrit la condensation du chlorure de l'acide trifluoroacétique avec $CH_2{=}C{=}0$ suivie d'une estérification avec l'éthanol. La condensation est effectuée à très basse température (— 30 °C) dans un milieu à base de dichloréthylène. Le chlorure de l'acide trifluoracétique est un gaz ayant un point d'ébullition très bas, difficilement disponible industriellement et toxique ; le cétène $CH_2{=}C{=}0$ est lui aussi dangereux à utiliser car il est instable et donc difficilement transportable. Ce procédé est donc difficilement inenvisageable du point de vue industriel.

Il a maintenant été découvert un nouveau procédé permettant de résoudre tous les problèmes de sécurité laissés par l'art antérieur. Selon ce procédé, dans une première étape, on effectue la condensation du trifluoroacétate d'éthyle (3) avec l'acétate d'éthyle (2) en présence d'éthanolate de sodium (1) dans le cyclohexane, dans une deuxième étape, on libère l'énol par l'acide formique (5), dans une troisième étape, on sépare le trifluoroacétoacétate d'éthyle par distillation.

L'éthanolate de sodium (1) peut être formé in situ par action directe du sodium sur l'éthanol dans le cyclohexane.

Le fait de réaliser la condensation en milieu anhydre, dans le cyclohexane permet d'éviter l'utilisation d'éther diéthylique dont l'emploi en grandes quantités est très délicate. Le cyclohexane est chimiquement neutre dans les conditions de réaction et il présente d'autre part l'avantage de former un azéotrope avec l'éthanol.

En effet, lors de la condensation, pour chaque mole de trifluoroacétoacétate d'éthyle formée, est consommée une mole d'éthanolate de sodium. Il se forme deux moles d'éthanol selon la réaction :

$$CH_3{-}COOC_2H_5 + C_2H_5ONa + CF_3COOC_2H_5 = CF_3{-}\underset{\displaystyle ONa}{\underset{|}{C}} = CH{-}COOC_2H_5 + 2C_2H_5OH \qquad (1)$$

L'éthanol est facilement éliminé du milieu par distillation sous forme d'azéotrope avec le cyclohexane (4). Cet azéotrope peut, soit être mis en contact avec du sodium métallique pour former de l'éthanolate de sodium qui sera recyclé au niveau de la première étape du procédé selon l'invention, soit être traité de manière connue, de façon à extraire l'alcool qui lui-même peut être recyclé vers la première étape.

Le produit issu de la première étape selon l'invention qui est un sel de sodium de l'énol de formule (I) est selon la deuxième étape du procédé de l'invention, neutralisé en milieu anhydre par un acide protonique, ce qui permet d'éviter l'hydrolyse provoquée par tous les acides forts aqueux de l'art antérieur. Cet acide doit, d'une part avoir un sel alcalin facilement précipitable en milieu organique, d'autre part, il doit avoir une acidité supérieure à celle de l'énol mais pas trop élevée pour ne pas dégrader le trifluoroacétoacétate d'éthyle.

L'acide formique répond à toutes ces conditions.

L'acide formique (5) est introduit en solution dans l'acétate d'éthyle.

On peut donner une composition préférée qui est une solution à 30 % en poids d'acide formique dans l'acétate d'éthyle.

Selon une mise en œuvre préférée de la deuxième étape du procédé de l'invention, après l'introduction de l'acide formique, on élimine l'acétate d'éthyle par une distillation de l'azéotrope (6)

acétate d'éthyle/cyclohexane.

Cet azéotrope est aisément recyclable dans la première étape du procédé.

Cette distillation azéotropique apporte en outre un avantage important car elle facilite la précipitation du sel de sodium de l'acide formique. Ce formiate de sodium (7) est éliminé par filtration ; le précipité est rincé plusieurs fois à l'aide de cyclohexane qui lui-même est joint au filtrat.

La troisième étape consiste à séparer le trifluoroacétoacétate de l'acétate d'éthyle, du trifluoroacétate d'éthyle et du cyclohexane subsistant. Il est évident que la quantité d'acétate d'éthyle à séparer sera beaucoup plus faible si, au cours de la deuxième étape préalablement à la filtration, on a effectué la distillation azéotropique acétate d'éthyle/cyclohexane.

Pour faciliter la séparation de ces différents constituants, il est particulièrement avantageux d'ajouter un solvant (8) qui permette de solubiliser les éléments lourds qui se sont formés au cours de la réaction. Ce solvant doit présenter un point d'ébullition supérieur à celui du trifluoroacétoacétate d'éthyle. L'orthodichlorobenzène répond à toutes ces exigences.

Selon un mode avantageux de mise en œuvre de la première étape selon l'invention, on préfère utiliser un rapport molaire du trifluoroacétate d'éthyle à l'éthanolate de sodium inférieur ou égal à 1, un rapport molaire de l'acétate d'éthyle au trifluoroacétate d'éthyle compris entre 1 et 2 et de préférence compris entre 1,5 et 2.

Selon un mode avantageux de mise en œuvre de la deuxième étape du procédé selon l'invention, on préfère utiliser un rapport molaire acide formique à l'éthanolate de sodium, d'environ 1.

Le trifluoroacétoacétate d'éthyle est utilisé comme intermédiaire de synthèse dans l'industrie pharmaceutique ou phytosanitaire (US 4251261, US 3953453).

L'invention va être plus complètement décrite à l'aide des exemples suivants qui ne doivent pas être considérés comme limitatifs de l'invention.

## Exemple 1

Dans un ballon de 10 litres surmonté d'une colonne de distillation de verre à plateaux (20 plateaux) de type « Older shaw », placé sous atmosphère d'azote sec, on prépare de l'éthylate de sodium de façon connue, à partir de 9,56 moles de Na (220 g) métallique et 5 180 g de EtOH absolu.

Après réaction totale de Na, on introduit des charges de cyclohexane en distillant chaque fois l'azéotrope alcool (cyclohexane), jusqu'à ce que la température en tête atteigne sensiblement le P.E. de cyclohexane pur (80-81 °C).

On introduit ainsi 17,6 litres de cyclohexane et récupère 21,8 litres d'azéotrope (16,7 kg).

Le milieu réactionnel est refroidi puis on introduit en 4 heures, tout en agitant, 9,56 moles (1 358 g) de trifluoroacétate d'éthyle pur.

Puis on introduit dans le milieu, à 50 °C, en 4 heures 30 mn, 19,12 moles d'acétate d'éthyle pur anhydre (1 687 g). On rajoute alors 3 litres de cyclohexane puis distille sous pression atmosphérique :

— tout d'abord une fraction de 2 822 g contenant 27 % de EtOH, 14 % de AcOEt, 1,25 % de trifluoroacétate d'éthyle, 57 % de cyclohexane, passant de 64 à 69 °C en tête

— puis une fraction de 1 181 g passant jusqu'à 77 °C de composition 3,83 % EtOH, 13,4 % de AcOEt, 82,8 % de cyclohexane (compositions par chromatographie phase vapeur).

— on charge de nouveau deux litres de cyclohexane puis écoule sous agitation, à 40 °C, un mélange de 440 g de HCOOH pur (9,56 moles) et de 1 026 g de AcOEt. Durée : 1 h 30 mn.

On laisse refroidir sous légère agitation. On filtre sur un appareil « Buchner » équipé d'une toile filtrante, puis rince une fois avec 1 litre de cyclohexane le précipité, puis une deuxième fois avec 0,75 litre.

— Le filtrat avec les rinçages au cyclohexane sont soumis à la distillation. On passe successivement :

sous pression atmosphérique, un mélange AcOEt/cyclohexane contenant quelques traces d'alcool, puis sous pression réduite à 140 mmHg, du cyclohexane presque pur (99,5 %), ensuite une fraction intermédiaire de 110 g à 75,5 % de TFAAE, passant en tête de colonne à la température de 35 °C,

sous pression réduite à 80 mmHg une masse de 1 197 g constituant la fraction principale, passant jusqu'à 66 °C.

Enfin, on réduit encore la pression à environ 50 mmHg et récupère une masse de distillat de 103 g. Il reste un résidu de 165 g, d'aspect goudronneux, devenant très visqueux (cireux) après refroidissement (température dans le bouilleur à la fin de la distillation : elle atteignait 142 °C).

On joint les trois dernières fractions distillées, soit 1 410 g, et soumet ce liquide à une nouvelle rectification avec une colonne de 20 plateaux, sous une pression de 140 mmHg. Avant d'atteindre une température fixe en tête de colonne de 82 °C à 83 °C, on sépare une masse de 32 g, puis on recueille à 82/83 °C une masse de 1 314 g de TFAAE pure à 99,1 % d'après l'analyse chromatographie phase vapeur.

On laisse un résidu de 58 g environ.

## Exemple 2

On utilise de l'éthylate de sodium solide (marque Dynamit Nobel) directement. Dans le même appareillage que précédemment, on introduit 1 700 g de cyclohexane et, sous atmosphère neutre (azote), 656 g d'éthylate technique solide, sous lente agitation. Puis comme dans l'exemple précédent, on

additionne le trifluoroacétate d'éthyle, puis l'acétate d'éthyle de la même façon et dans les mêmes quantités. On poursuit de la même manière que dans l'exemple précédent jusqu'à la filtration du formiate de sodium et le rinçage du gâteau sur filtre.

On ajoute alors 625 g d'orthodichlorobenzène au liquide obtenu, avant de le soumettre à la distillation.

Après séparations successives du binaire AcOEt/cyclohexane, puis du reste du cyclohexane sous pression réduite à 140 mmHg, on distille d'abord sous cette pression, puis en la réduisant à 95 mmHg, une masse de 1 321 g contenant, d'après l'analyse chromatographie phase vapeur, 98,8 % de trifluoroacétoacétate d'éthyle. A la fin de la distillation, la température dans le bouilleur ne dépasse pas 115 °C. On écoule facilement le résidu liquide à froid, d'une masse de 780 g.

**Revendications**

1. Procédé de préparation de trifluoroacétoacétate d'éthyle caractérisé en ce que dans une première étape, on effectue la condensation du trifluoroacétate d'éthyle (3) et de l'acétate d'éthyle (2) en présence d'éthanolate de sodium (1) dans le cyclohexane, que dans une deuxième étape, on libère l'énol par l'acide formique (5), que dans une troisième étape, on sépare le trifluoroacétoacétate d'éthyle par distillation.

2. Procédé selon la revendication 1 caractérisé en ce que lors de la première étape, l'éthanolate de sodium est formé in situ par action du sodium métallique sur l'éthanol.

3. Procédé selon la revendication 1 caractérisé en ce que lors de la première étape après la condensation du trifluoroacétate d'éthyle et de l'acétate d'éthyle, on distille l'éthanol libéré sous forme d'un azéotrope (4) avec le cyclohexane.

4. Procédé selon la revendication 1 caractérisé en ce que lors de la deuxième étape, l'acide formique est utilisé en solution dans l'acétate d'éthyle.

5. Procédé selon la revendication 1 caractérisé en ce que lors de la deuxième étape, le formiate de sodium (7) formé lors de l'énolisation est éliminé par filtration.

6. Procédé selon la revendication 1 caractérisé en ce que avant la filtration, on élimine l'acétate d'éthyle par distillation d'un azéotrope (6) avec le cyclohexane.

7. Procédé selon les revendications 1 et 5 caractérisé en ce que lors de la troisième étape, on sépare par distillation le trifluoroacétoacétate formé, le cyclohexane et éventuellement l'acétate d'éthyle.

8. Procédé selon la revendication 7 caractérisé en ce que préalablement à la distillation, on ajoute de l'orthodichlorobenzène (8).

9. Procédé selon la revendication 3 caractérisé en ce que l'azéotrope éthanol/cyclohexane (4) est recyclé vers la première étape.

10. Procédé selon la revendication 9 caractérisé en ce que l'azéotrope acétate d'éthyle/cyclohexane (6) est recyclé vers la première étape.

**Claims**

1. Process for preparing ethyl trifluoroacetoacetate, characterized in that, in a first stage, the condensation of ethyl trifluoroacetate (3) and ethyl acetate (2) is performed in the presence of sodium ethanolate (1) in cyclohexane, in that, in a second stage, the enol is liberated with formic acid (5), and in that, in a third stage, the ethyl trifluoroacetoacetate is separated by distillation.

2. Process according to claim 1, characterized in that, during the first stage, the sodium ethanolate is formed in situ by the action of sodium metal on ethanol.

3. Process according to claim 1, characterized in that, during the first stage, after the condensation of the ethyl trifluoroacetate and the ethyl acetate, the ethanol liberated is distilled in the form of an azeotrope (4) with cyclohexane.

4. Process according to claim 1, characterized in that, during the second stage, the formic acid is used in solution in ethyl acetate.

5. Process according to claim 1, characterized in that, during the second stage, the sodium formate (7) formed during the enolization is removed by filtration.

6. Process according to claim 1, characterized in that, before the filtration, the ethyl acetate is removed by distillation of an azeotrope (6) with cyclohexane.

7. Process according to claims 1 and 5, characterized in that, during the third stage, the trifluoroacetoacetate formed, the cyclohexane and, where appropriate, the ethyl acetate are separated by distillation.

8. Process according to claim 7, characterized in that, prior to the distillation, ortho-dichlorobenzene (8) is added.

9. Process according to claim 3, characterized in that the ethanol/cyclohexane azeotrope (4) is recycled to the first stage.

10. Process according to claim 9, characterized in that the ethyl acetate/cyclohexane azeotrope (6) is recycled to the first stage.

**Patentansprüche**

1. Verfahren zur Herstellung von Ethyl-trifluoracetoacetat, dadurch gekennzeichnet, daß man in einer ersten Stufe die Kondensation des Ethyl-trifluoracetats (3) und des Ethylacetats (2) in Gegenwart von Natriumethanolat (1) in Cyclohexan durchführt, daß man in einer zweiten Stufe das Enol durch Ameisensäure (5) freisetzt, und daß man in einer dritten Stufe das Ethyl-trifluoracetoacetat durch Destillation abtrennt.

2. Verfahren nach Anspruch 1, dadurch gekennzeichnet, daß bei der ersten Stufe das Natriumethanolat in situ durch Einwirkung von metallischem Natrium auf Ethanol gebildet wird.

3. Verfahren nach Anspruch 1, dadurch gekennzeichnet, daß man bei der ersten Stufe, nach der Kondensation des Ethyl-trifluoracetats mit dem Ethylacetat, das freigesetzte Ethanol in Form eines Azeotrops (4) mit Cyclohexan abdestilliert.

4. Verfahren nach Anspruch 1, dadurch gekennzeichnet, daß die Ameisensäure in der zweiten Stufe in Lösung mit Ethylacetat eingesetzt wird.

5. Verfahren nach Anspruch 1, dadurch gekennzeichnet, daß das Natriumformiat (7), das während der Enolisierung gebildet wird, bei der zweiten Stufe durch Filtration entfernt wird.

6. Verfahren nach Anspruch 1, dadurch gekennzeichnet, daß man vor der Filtration das Ethylacetat durch Destillation eines Azeotrops (6) mit Cyclohexan entfernt.

7. Verfahren nach den Ansprüchen 1 bis 5, dadurch gekennzeichnet, daß man bei der dritten Stufe durch Destillation das gebildete Trifluoracetoacetat, das Cyclohexan und gegebenenfalls das Ethylacetat durch Destillation abtrennt.

8. Verfahren nach Anspruch 7, dadurch gekennzeichnet, daß man vor der Destillation ortho-Dichlorbenzol (8) zusetzt.

9. Verfahren nach Anspruch 3, dadurch gekennzeichnet, daß das Ethanol/Cyclohexan-Azeotrop (4) in die erste Stufe zurückgeführt wird.

10. Verfahren nach Anspruch 9, dadurch gekennzeichnet, daß das Ethanol/Cyclohexan-Azeotrop (6) in die erste Stufe zurückgeführt wird.

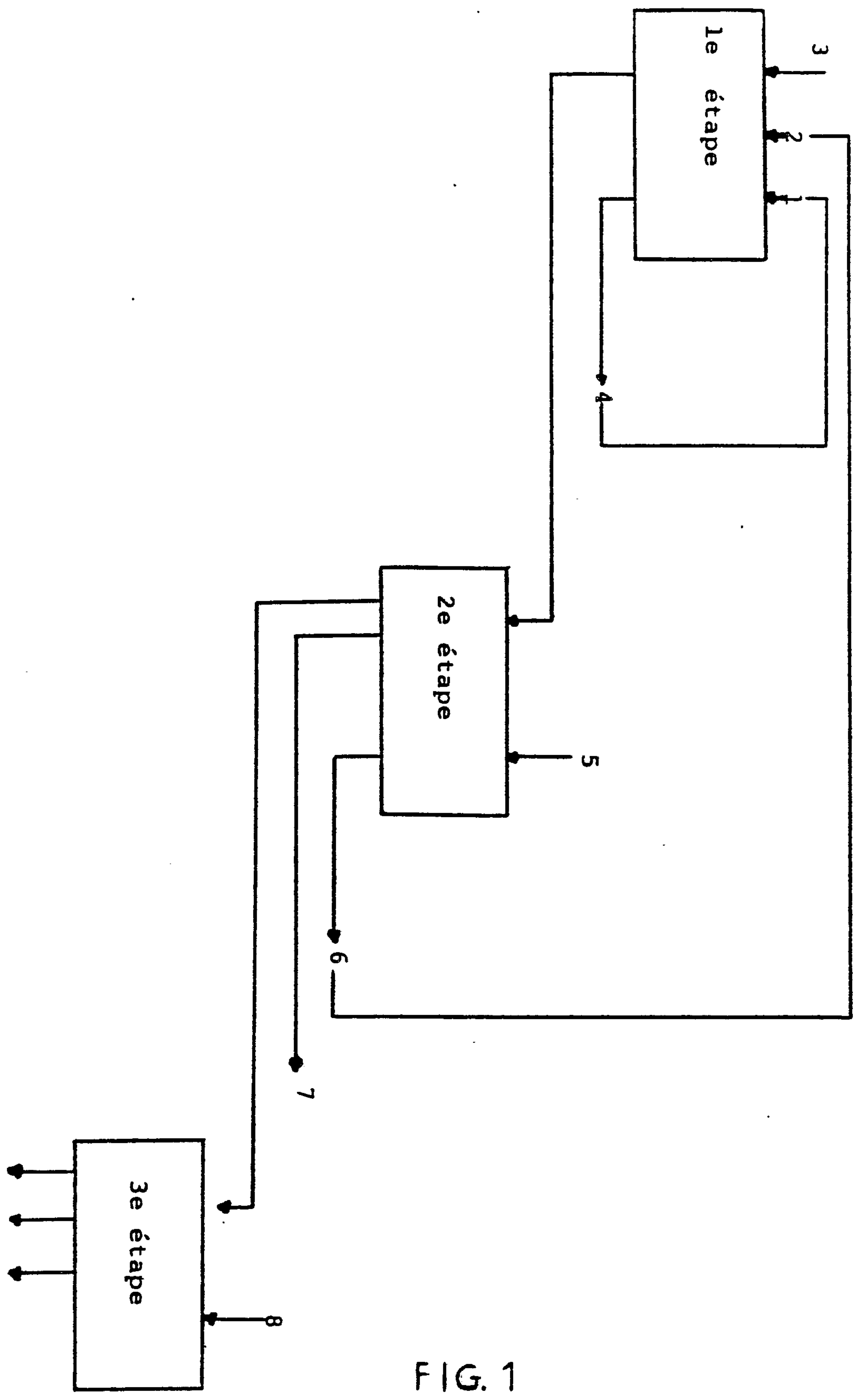


FIG. 1